# EUROPEAN PATENT APPLICATION

(11) **EP 2 977 001 A1**
(43) Date of publication of application: **27.01.2016**
(21) Application number: 13879127.2
(22) Date of filing: 28.11.2013
(51) Int. Cl.: A61B 5/055, G01R 33/385, G01R 33/48, G01R 33/28

(54) **NUCLEAR MAGNETIC RESONANCE IMAGING SYSTEM AND METHOD**

(30) Priority: 20.03.2013 CN 201310090452
(71) Applicant: Jiangsu Magspin Instrument Co., Ltd, Kunshan, Jiangsu 215300 (CN)
(72) Inventor: ZHAO, Huawei, Kunshan Jiangsu 215300 (CN)
(74) Representative: Tischner, Oliver
(86) International application number: PCT/CN2013/088061
(87) International publication number: WO 2014/146449

(57) **Abstract**

A nuclear magnetic resonance imaging system and imaging method. The system includes: an independently arranged main magnet (30); and a gradient coil (40) and a radio frequency coil (50), both connected to a patient bed (60). A bore (100) is formed in a central portion of the main magnet (30), for containing the patient bed (60), the gradient coil (40) and the radio frequency coil (50). In this system, the gradient coil (40) and the radio frequency coil (50) are separated from the main magnet (30) instead of being integrated with the main magnet (30), thus avoiding making a patient claustrophobic feeling in a too narrow space. In addition, the system lowers the requirements on the magnet material and shielding space and thus leads to further reductions in costs and maintenance expenses.

## Description

### TECHNICAL FIELD

The present invention relates to a magnetic resonance imaging (MRI) system incorporating an independently arranged main magnet and imaging method.

### BACKGROUND

Magnetic resonance imaging (MRI) systems are often used in the field of medical health care. Hydrogen atomic nuclei (protons) in body tissues can release nuclei magnetic resonance signals when excited by radio frequency pulses in a magnetic field. MRI is an imaging technique which allows computerized reconstruction of cross-sectional body image slices from the nuclei magnetic resonance signals.

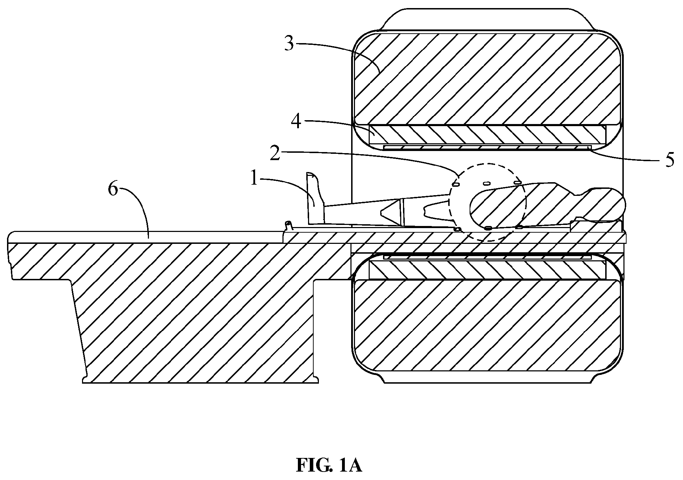

During the use of MRI, as shown in FIGs. 1A to 1B, the body 1 of a patient (or an object of interest) is positioned in an imaging region 2 which is defined by the design and positioning arrangement of the MRI system. A conventional MRI system as an integrated design includes, from the outside inward, a main magnet 3, gradient coils 4 and radio frequency coil 5 arranged at fixed positions, in which the imaging region 2 is typically a central region of a central bore defined in the main magnet 3, and the imaging region 2 is non-adjustable in terms of both position and size. Such a system as an integrated design is problematic in which the central bore where the body 1 of the patient, as well as a patient bed 6, is positioned within a very limited space which tends to make patient claustrophobic feeling. In addition, in order to obtain a clear image of a body 1 portion of the patient of interest, the portion must be properly positioned in the imaging region 2, which is, however, a very challenging task to be fulfilled in the limited space in case of the portion being a limb portion such as shoulder. This will makes the imaging of the limb portion very difficult and even impossible when the patient has a large body size.

Therefore, there is an urgent need in this art for an MRI system and method which allow the body of a patient to be positioned in a larger space and enable a flexible imaging region.

### SUMMARY OF THE INVENTION

The present invention seeks to expand the central bore for containing the body of a patient so as to allow a body portion of the patient of interest to be positioned in an imaging region in a more comfortable and flexible manner by presenting a magnetic resonance imaging (MRI) system and method.

In pursuit of this aim, the invention provides an MRI system which includes: a main magnet, provided with a bore formed in a central portion thereof; a patient bed, movable in and out of the bore; a gradient coil and a radio frequency coil, both connected to the patient bed and separated from the main magnet.

Preferably, the MRI system further includes field shimming coil which is connected to the patient bed.

Preferably, the gradient coil, the radio frequency coil and the field shimming coils are all in detachable or movable connection within the patient bed.

Preferably, the radio frequency coil, the gradient coil and the field shimming coils means sequentially cover a portion of interest of the patient body and each have a size and a shape conforming with a size and a shape of the body portion of interest.

Preferably, the main magnet is a unitary formed cylinder with an internal bore.

The invention also provides an MRI method including the steps of:
providing a main magnet having a bore thereof;
disposing a body of a patient on a patient bed;
connecting a gradient coil and a radio frequency coil, which are both separated from the main magnet, to the patient bed and disposing the gradient coil and the radio frequency coil over a portion of interest of the body of the patient; and
moving the patient bed into the bore and performing MRI on the portion of interest of the body of the patient.

Compared to the conventional MRI system, the MRI system and method according to the present invention have a number of advantages as follows:
1. substitution of the conventional structure in which the main magnet, the gradient coil and the radio frequency coil are integrated together with the inventive structure in which the gradient coil and the radio frequency coil are separated from the main magnet enabling the main magnet to provide both the patient and an operator with a larger space which can make a patient with claustrophobia feel relaxed and enabling new applications of MRI such as, for example, imaging of a moving body which requires a larger space;
2. it also allows a shrinkage in the size of the main magnet and a significant reduction in the 5-Gauss line while providing the patient with the same size of imaging region, thereby resulting in reductions in costs for the magnet and shielding space; and
3. the inventive smaller main magnet can lead to less concern about weight increasing in high-field applications and less coolant consumption at even higher magnetic field strengths.

### BRIEF DESCRIPTION OF THE DRAWINGS

FIG. 1A is an elevation cross-sectional view of a conventional magnetic resonance imaging (MRI) system.
FIG. 1B is a side view of the conventional MRI system.
FIG. 2 is a side view of an MRI system in accordance with an embodiment of the present invention.
FIG. 3 is a schematic illustration of an MRI system in accordance with a specific embodiment of the present invention (in use of head imaging).
FIG. 4A to 4B schematically illustrate an MRI system in accordance with a specific embodiment of the present invention (in use of foot imaging).
FIG. 5A to 5B schematically illustrate an MRI system in accordance with a specific embodiment of the present invention (in use of shoulder imaging).

### DETAILED DESCRIPTION

In order to describe the above aspects of the present invention in greater detail, specific embodiments are set forth below to demonstrate the advantages of the invention. It is to be noted that these embodiments are illustrative only and not intended to be limiting of the scope of the present invention.

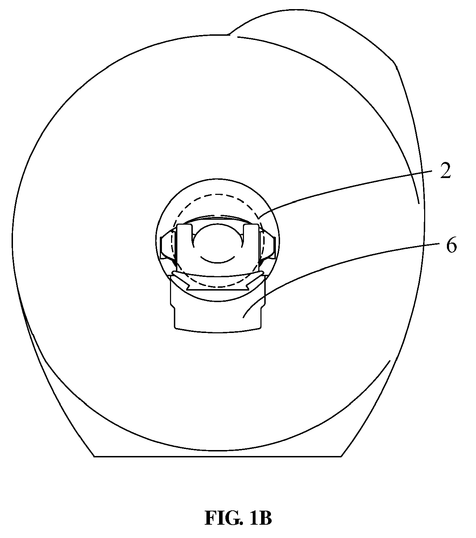

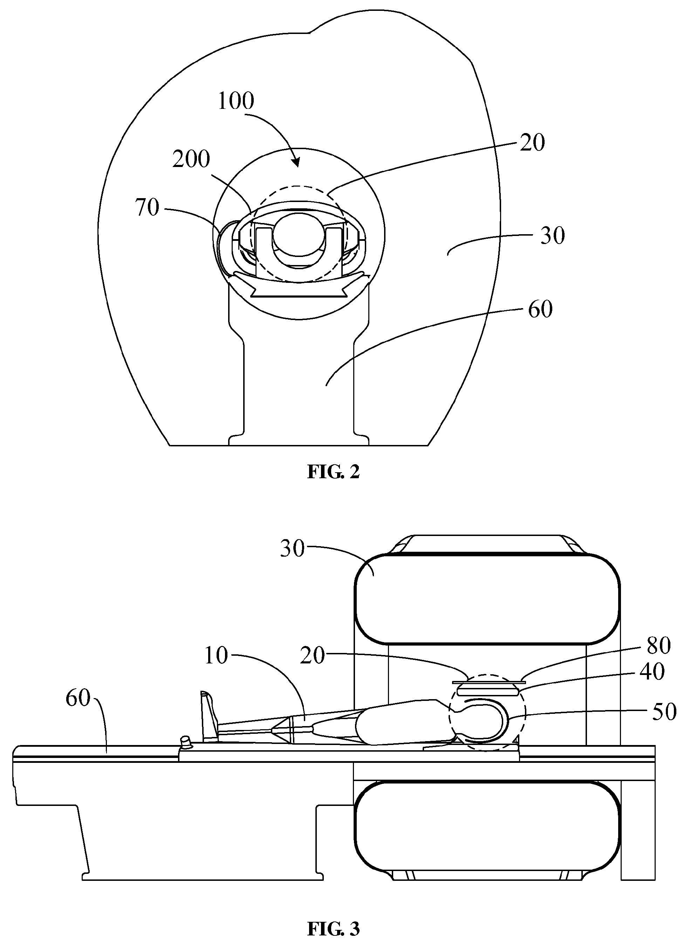

A magnetic resonance imaging (MRI) system constructed in accordance with the present invention, as shown in FIG. 2, includes an independently arranged main magnet 30 and a coil assembly 200 for adjusting a magnetic field. The coil assembly 200 is connected to a patient bed 60 by a cable 70. In a central portion of the main magnet 30, a bore 100 is formed for receiving the patient bed 60 and the coil assembly 200. According to an embodiment, the coil assembly 200 includes a gradient coil and a radio frequency coil. Compared to the conventional system (FIG. 1A) in which the main magnet 3, the gradient coil 4 and the radio frequency coil 5 are integrated together, in the system according to the present invention, the gradient coil 40 and radio frequency coil 50 are separated from the main magnet 30. From a comparison between FIG. 1B illustrating the conventional system and FIG. 2 illustrating the inventive system, it is readily understandable that, for the same size of imaging region, the inventive system allows the bore 100 in the main magnet 30 to have an inner diameter of 700-900 mm, compared to an inner diameter of 600 mm in the conventional system. This avoids making a patient feel claustrophobic in a too narrow space. Additionally, the present invention eliminates the need to employ a radio frequency coil disposed over the whole length of the main magnet 30 for performing radio frequency detection over the whole body of the patient, thereby ensuring that the power absorption per unit mass of tissue is lower than the safe limit.

Preferably, with continuing reference to FIGs. 2 and 3, the coil assembly 200 may further include a field shimming means 80 which may also be connected to the patient bed 60 via the cable 70. The radio frequency coil 50, together with the main magnet 30, produces magnetic field. The gradient coil 40 can augment or attenuate intensities in the magnetic field produced by the main magnet 30 such that spinning protons distributed along a gradient are affected by different magnetic field strengths and hence resonate at different frequencies. The field shimming means 80 is configured to homogenize the magnetic field.

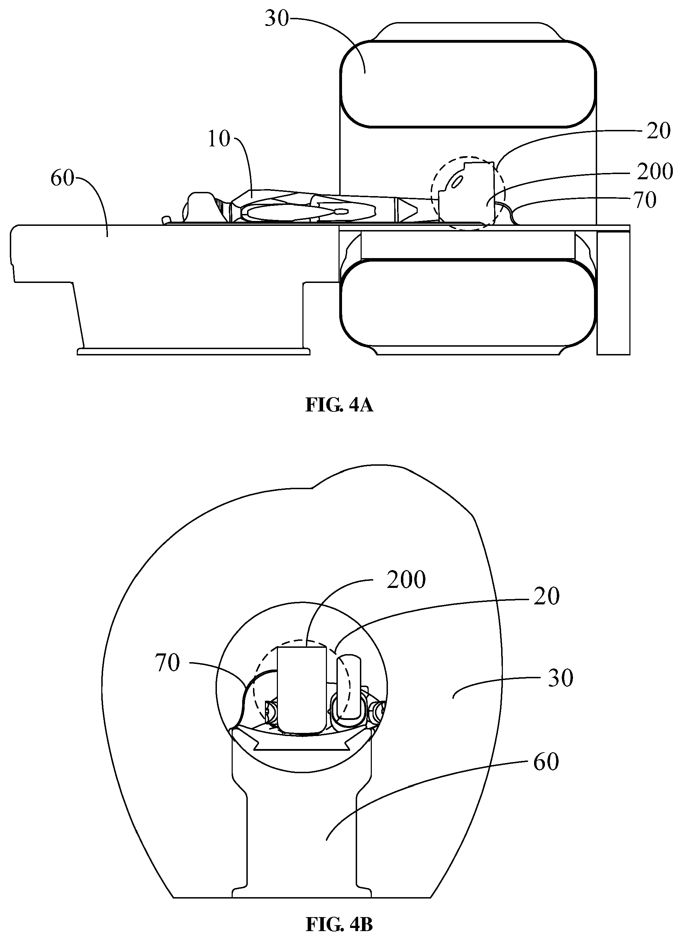

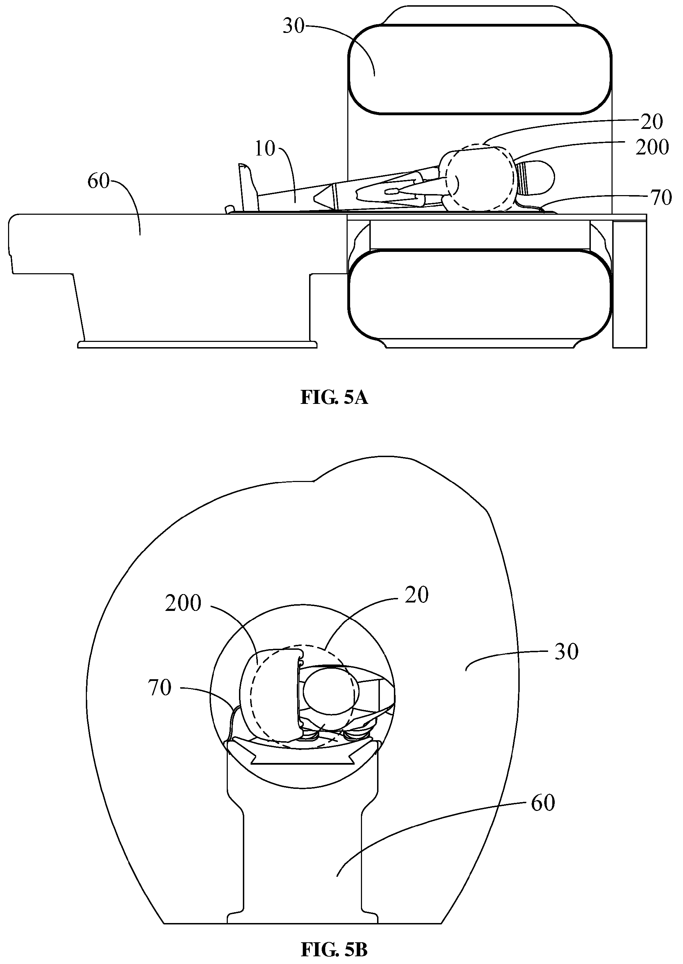

Preferably, with continuing reference to FIGs. 2 and 3, the gradient coil 40, the radio frequency coil 50 and the field shimming means 80 are all in detachable or movable connection with the patient bed 60, and the gradient coil 40, the radio frequency coil 50 and the field homogenization means 80 are sequentially disposed over a portion of interest of the body of a patient. Preferably, each of the gradient coil 40, the radio frequency coil 50 and the field homogenization means 80 has a size and a shape conforming to a size and a shape of the portion of interest of the body of the patient. For example, the gradient coil 40, the radio frequency coil 50 and the field shimming means 80 may each have a size and a shape adapted to the profile of a head when in use of head imaging, as shown in FIG. 3. Or, the gradient coil 40, the radio frequency coil 50 and the field shimming means 80 may each have a size and a shape adapted to the profile of a foot when in use of foot imaging, as shown in FIGs. 4A to 4B. Or, the gradient coil 40, the radio frequency coil 50 and the field shimming means 80 may each have a size and a shape adapted to the profile of a shoulder when in use of shoulder imaging, as shown in FIGs. 5A to 5B. For the sake of simplicity, the gradient coil 40, the radio frequency coil 50 and the field shimming means 80 are depicted in FIGs. 4A to 4B and FIGs. 5A to 5B as an integral structure, i.e., the coil assembly 200. As such, use of the gradient coil 40, the radio frequency coil 50 and the field shimming means 80 that are smaller-sized and locally adaptable to various portions of the patient body can provide the advantages as follows: the locally applied gradient coil 40 produces a highly linear magnetic field gradient in a smaller space, thus reducing physiological restrictions to the magnetic field rate (dB/dt) in terms of safety and achieving efficient gradient utilization in high-field MRI; as the gradient coil 40 is subject to a mechanical force that is proportional to the magnetic field strength that it experiences, separation of the gradient coil 40 from the main magnet 30 according to the present invention can result in a significant reduction in acoustic noise compared to the conventional system, which can increase patient comfort and mechanic robustness, particularly in high-field MRI application; the locally applied radio frequency coil 50 and field shimming means 80 can effectively compensate for magnetic effects additionally induced by increasing in magnetic field strength and eliminating the need for a radio frequency coil 50 disposed over the whole patient body, and the radio frequency coil 50 separated from the main magnet 30 can be better compatible with the shape of the portion of interest and thus better control the specific absorption rate (SAR), a measure of the rate at which energy is absorbed by the human body, to a level under the safety limit; and the main magnet 30, the gradient coil 40 and the radio frequency coil 50 have the following advantage in terms of engineering: they can effectively reduce some basic restrictions to high-field applications of MRI, such as safety, power consumption and maintenance cost, thereby reducing the effort to balance various engineering considerations and allowing the development of new techniques and hardware on the basis of new pulse sequence designs. Therefore, the present invention can achieve high space flexibility while ensuring good imaging performance.

It is to be noted that, according to the present invention, the gradient coil 40, the radio frequency coil 50 and the field shimming means 80 are not limited to the structures and shapes that are presented in the accompanying drawings only for the purpose of illustration. That is, the gradient coil 40, the radio frequency coil 50 and the field shimming means 80 are all deployed with the patient bed 60 serving as a support and the deployment may be accomplished in any suitable manner that allows them to work together with the main magnet 30 to perform MRI.

Preferably, with continuing reference to FIG. 2, the main magnet 30 is a unitary hollow cylinder. According to the present invention, before the patient bed 60 is moved into the bore 100 formed in the central portion of the main magnet 30, a clinician is allowed to adjust the positions of the gradient coil 40, the radio frequency coil 50 and the field shimming means 80 to make them better fitting the body 10 of the patient and thus obtain a larger activity space of the clinician.

Further, as shown in FIGs. 4B and 5B, the expansion of the inner diameter of the bore 100 in the main magnet 30 results in additional space, where the patient can move the body in while lying on the patient bed 60. The MRI system according to the present invention can image some limb portions that are barely handled by the conventional MRI system, such as foots and shoulders, and allows a body portion of interest to be positioned in the imaging region 20 in a more comfortable and flexible way.

With reference to FIGs. 2 to 3, 4A to 4B and 5A to 5B, an MRI method according to the present invention for use in the above described MRI system includes the steps of:
S1) disposing the body of the patient 10 on the patient bed 60;
S2) positioning the gradient coil 40 and the radio frequency coil 50, of course, as well as the field shimming means 80, over a portion of interest of the body 10 of the patient; and
S3) moving the patient bed 60 into the bore 100 formed in the central portion of the main magnet 30 and performing MRI on the portion of interest.

In summary, the present invention provides an MRI system and an MRI method. The system includes: an independently arranged main magnet 30; and a gradient coil 40 and a radio frequency coil 50, both connected to a patient bed 60. A bore 100 is formed in a central portion of the main magnet 30, for receiving the patient bed 60, the gradient coil 40 and the radio frequency coil 50. Compared to the conventional structure in which the main magnet, the gradient coil and the radio frequency coil are integrated together, the structure according to the present invention in which the gradient coil 40 and the radio frequency coil 50 are separated from the main magnet 30 avoids making a patient claustrophobic feeling in a too narrow space. In addition, the present invention lowers the requirements on the magnet material and shielded space associated with the use of the conventional MRI system and thus leads to further reductions in related costs and maintenance expenses.

Obviously, those skilled in the art may make various modifications and alterations without departing from the spirit and scope of the invention. It is therefore intended that the invention be construed as including all such modifications and alterations insofar as they fall within the scope of the appended claims or equivalents thereof.

## Claims

1. A magnetic resonance imaging (MRI) system, comprising:
a main magnet, provided with a bore formed in a central portion thereof;
a patient bed, movable in and out of the bore;
a gradient coil and a radio frequency coil, both connected to the patient bed and separated from the main magnet.

2. The MRI system of claim 1, further comprising a field shimming means connected to the patient bed.

3. The MRI system of claim 2, wherein the gradient coil, the radio frequency coil and the field homogenization means are all in detachable or movable connection with the patient bed.

4. The MRI system of claim 2, wherein the gradient coil, the radio frequency coil and the field shimming means are sequentially disposed over a portion of interest of a body of a patient and each have a size and a shape corresponding to a size and a shape of the portion of interest.

5. The MRI system of claim 1, wherein the main magnet is a unitary hollow cylinder and the bore is a circular bore.

6. A magnetic resonance imaging (MRI) method comprising:
providing a main magnet having a bore formed in a central portion thereof;
disposing a body of a patient on a patient bed;
connecting a gradient coil and a radio frequency coil, which are both separated from the main magnet, to the patient bed and disposing the gradient coil and the radio frequency coil over a portion of interest of the body of the patient; and
moving the patient bed into the bore and performing MRI on the portion of interest of the body of the patient.

7. The method of claim 6, further comprising:
connecting a field shimming means to the patient bed and positioning the field shimming means on the portion of interest of the body of the patient.

8. The method of claim 7, wherein the radio frequency coil, the gradient coil and the field shimming means are sequentially disposed over the portion of interest of the body of the patient.

9. The method of claim 6, wherein the main magnet is a unitary hollow cylinder and the bore is a circular bore.
